# EUROPEAN PATENT SPECIFICATION

(11) **EP 3 054 924 B1**
(45) Date of publication and mention of the grant of the patent: **25.11.2020**
(21) Application number: 14780863.8
(22) Date of filing: 06.10.2014
(51) Int. Cl.: A61K 9/19, A61K 38/12

(54) **STABLE PHARMACEUTICAL FORMULATIONS OF CASPOFUNGIN**
STABILE PHARMAZEUTISCHE FORMULIERUNGEN VON CASPOFUNGIN
FORMULATIONS PHARMACEUTIQUES STABLES DE CASPOFUNGINE

(30) Priority: 07.10.2013 EP 13187612; 20.02.2014 EP 14155993; 27.03.2014 EP 14162110
(43) Date of publication of application: 17.08.2016
(73) Proprietor: Galenicum Health S.L.U., 08005 Barcelona (ES); Centrient Pharmaceuticals Netherlands B.V., 2613 AX Delft (NL)
(72) Inventor: CUBEL SUÑÉ, Núria, E-08005 Barcelona (ES)
(74) Representative: Pons Ariño, Angel
(86) International application number: PCT/EP2014/071302
(87) International publication number: WO 2015/052115

(56) References cited:
- WO-A1-2008/012310
- WO-A1-2009/002481
- WO-A1-2012/038371

## Description

The present invention relates to a stable pharmaceutical composition comprising caspofungin and a buffering agent selected from the group consisting of carbonic acid, bicarbonate, carbon dioxide and carbonate, to a process for the manufacture of said pharmaceutical composition as well as to its use in the treatment of fungal infections and/or conditions arising from said infections.

### STATE OF THE ART

Caspofungin (CAS 162808-62-0) is the first of a new class of semi-synthetic antifungal agents belonging to the class of echinocandins which is known to be an effective antifungal and anti-protozoal agent. It is represented by the formula (I):

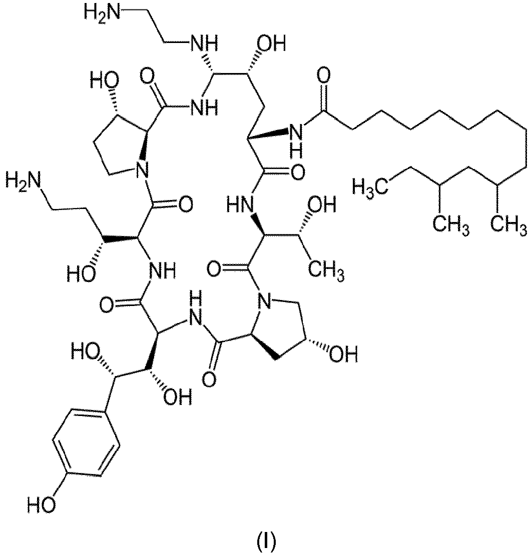

Caspofungin is effective in the prevention and/or treatment of infections caused by filamentous fungi and yeasts such as *Aspergillus sp., Histoplasma sp., Coccidioides sp., Blastomyces sp.* and/or *Candida sp.,* as well as in preventing and/or controlling and/or treating infections such as pneumonia caused by *Pneumocystis jiroveci* (previously classified as *Pneumocystis carinii).* Caspofungin may be administered parenterally, e.g. intravenously by use of compositions being either lyophilized or liquid formulations.

A lyophilized caspofungin product is available on the market as its diacetate salt under the trade name Cancidas® (RLD product). Cancidas® contains, in addition to the active ingredient caspofungin diacetate, acetic acid, sodium hydroxide, sucrose and mannitol. Before administration to a patient, the lyophilized product is reconstituted by adding a diluent and the desired amount of the diluted mixture is transferred to an infusion bag to be administered to the patient in need thereof.

A well-known problem with caspofungin compositions prepared for reconstitution prior to administration to the patient, is that the compound is highly unstable resulting in the formation of various degradation products such as e.g. hydrolysis products (impurity B) and dimerization products (impurity C). There will also be impurities present in the composition being formed during the fermentation of the starting material and which have passed along through the synthesis of caspofungin. The main impurity originating from the fermentation is the serine analogue of caspofungin having the formula as shown in WO 2009/158034.

In addition to the above-mentioned degradation impurities and impurities formed during preparation of known caspofungin compositions, further non-characterised impurities are also present. The mechanisms behind the formation of the impurities are not fully understood. However, it is known that the buffer system used when preparing the composition may increase the degradation product formation during preparation and storage. In US 5,952,300 it is e.g. stated that the use of tartrate buffer resulted in undesired degradation products. The solution to said degradation problem according to the teaching of US 5,952,300 is the use of an acetate buffer.

Various other strategies are also known to avoid degradation and improve the stability of caspofungin compositions. For example, WO 2009/002481 discloses a lyophilized caspofungin composition comprising, in addition to caspofungin diacetate and an acetate buffer, one or more non-reducing sugars such as trehalose, sucrose, raffinose, or sorbitol or combinations thereof.

In WO 2008/012310, a caspofungin composition is disclosed comprising, in addition to a pharmaceutically acceptable salt of caspofungin and excipients, only very low levels of a buffering agent, or which is free of a buffering agent.

WO 2012/038371 discloses a composition comprising caspofungin or a pharmaceutically acceptable salt thereof, one or more excipients effective to form a lyophilized cake, and a buffering agent selected from the group consisting of lactate and succinate.

Although various solutions to the impurity problem are suggested in the prior art, there is still a need for a caspofungin composition with improved stability in respect of the formation of impurities during storage. Furthermore, it would be desirable to provide compositions having a good stability and a long shelf life and which may be manufactured by a simple and fast method.

### SUMMARY OF THE INVENTION

The inventors have surprisingly found that an antifungal composition according to the present invention comprising caspofungin or a pharmaceutically acceptable salt thereof, one or more pharmaceutically acceptable excipients and including a buffering agent selected from the group consisting of carbonic acid, bicarbonate, carbon dioxide, carbonate and mixtures thereof, is stable, resulting in low formation of degradation products during storage.

The present inventors have found that the addition of a buffering agent selected from the group consisting of carbonic acid, bicarbonate, carbon dioxide, carbonate and mixtures thereof is sufficient to obtain a stable formulation. The resulting formulation contains a low percentage of total impurities.

The present invention therefore provides a pharmaceutical composition comprising a) a pharmaceutically effective amount of caspofungin or a pharmaceutically acceptable salt thereof; b) a pharmaceutically acceptable amount of one or more excipients effective to form a cake; c) a pharmaceutically acceptable amount of a buffering agent selected from the group consisting of carbonic acid, bicarbonate, carbon dioxide, carbonate and mixtures thereof.

Advantageously, lyophilized preparations are stable, can be stored and are easily reconstituted. Moreover, lyophilized preparations may be kept sterile and essentially free of insoluble matter.

In a first aspect, it is provided a pharmaceutical composition comprising:
a) a pharmaceutically effective amount of caspofungin and/or a pharmaceutically acceptable salt thereof;
b) a pharmaceutically acceptable amount of one or more excipients effective to form a cake;
c) a pharmaceutically acceptable amount of a buffering agent selected from the group consisting of carbonic acid, bicarbonate, carbon dioxide, carbonate and mixtures thereof.

In a second aspect, it is provided a lyophilized cake obtainable by lyophilization of the composition of the first aspect in form of a solution, said lyophilized cake is suitable for reconstitution to form a liquid composition for parenteral, preferably intravenous, administration.

In a third aspect, it is provided a pharmaceutical composition obtainable by reconstitution of the lyophilized cake of the second aspect with a sterile aqueous solution.

In a fourth aspect, it is provided a process for the manufacture of a pharmaceutical composition as herein disclosed, comprising the following steps:
i) preparing an aqueous solution comprising a pharmaceutically acceptable amount of one or more excipients with a pharmaceutically acceptable amount of the buffering agent;
ii) adding to the solution of (i) a pharmaceutically acceptable amount of caspofungin and/or a pharmaceutically acceptable salt thereof;
iii) optionally, adjusting the pH between 4 and 7;
iv) filtering the solution obtained in the previous step;
v) freezing the solution obtained in step (iv); and
vi) freeze drying the frozen solution obtained in step (v).

In a fifth aspect, it is provided a pharmaceutical composition obtained by the process of the fourth aspect.

In a sixth disclosure, it is provided a pharmaceutical batch of the pharmaceutical composition as herein disclosed.

In a seventh disclosure, it is provided a pharmaceutical batch comprising at least 20 % w/w of caspofungin diacetate in respect of the total amount of the batch and wherein the molar ratio of the pharmaceutically effective amount of caspofungin and/or a pharmaceutically acceptable salt thereof and the buffering agent is from 10:1 to 1:5.

In an eighth aspect, it is provided a sealed glass vial comprising the pharmaceutical formulation as herein disclosed.

In a ninth disclosure, it is provided a cardboard box with a patient information leaflet comprising at least one unit of the pharmaceutical composition as herein disclosed.

In a tenth disclosure, it is provided the use of the composition as herein disclosed for the manufacture of a medicament, preferably an intravenous medicament, for the prevention and/or treatment of fungal infections or conditions caused by *Candida* species and /or *Aspergillus* species.

In an eleventh aspect, it is provided a process for the manufacture of a lyophilized cake comprising caspofungin or a pharmaceutically acceptable salt thereof, wherein the process of freeze drying is less than 96 hours long, preferably less than 72 hours long, and more preferably less than 48 hours long from the initial step of freezing the solution until the end of the secondary drying, wherein the process comprises at least the following steps:
i) freezing a suitable solution to a first temperature, wherein the first temperature is a temperature between -40 °C and -50 °C, and holding for between at least 3 hours to 8 hours at the first temperature;
ii) primary drying between 20 to 40 hours under vacuum from the first temperature to a second temperature, wherein the second temperature is between 10 °C and 0 °C;
iii) secondary drying for at least 4 hours under vacuum from the second temperature to a third temperature, wherein the third temperature is between +35 °C to +25 °C..

Another aspect of the present invention relates to the pharmaceutical composition or the glass vial as herein disclosed for use in the treatment of fungal infections or conditions caused by *Candida* species and/or *Aspergillus* species.

### DETAILED DESCRIPTION OF THE INVENTION

The present disclosure provides a highly stable formulation both in lyophilised form and as a solution after reconstitution of the lyophilized cake.

The term "stable" as used herein refers to a pharmaceutical composition comprising caspofungin diacetate wherein the total content of impurities originating from the decomposition of caspofungin diacetate does not exceed 5 % area, preferably 3 % area and more preferably 2 % area determined by liquid chromatography (HPLC) at 210 nm if such a composition is stored for 6 months at 5 °C. Further, the term "stable" as used herein for lyophilized forms, refers to a pharmaceutical composition which when reconstituted in a sterile liquid vehicle, the reconstituted solution is clear without precipitation of the water insoluble drug for at least 2 hours after addition of the sterile liquid vehicle.

The term "active ingredient" refers to a therapeutically active compound, as well as any prodrugs thereof and pharmaceutically acceptable salts, hydrates and solvates of the compound and the prodrugs.

The term "pharmaceutically acceptable" indicates that the substance or composition must be compatible chemically and/or toxicologically, with the other ingredients comprising a formulation, the mammal being treated therewith, and/or the route of administration of the composition.

In a further embodiment of the pharmaceutical composition of the first aspect, the pharmaceutical composition comprises between 25 and 80 mg of caspofungin as free base by unit dose.

In a further embodiment of the pharmaceutical composition as herein disclosed, the pharmaceutical composition comprises about 50 mg of caspofungin as free base by unit dose or comprises about 70 mg of caspofungin as free base by unit dose.

As used herein, the term "unit dose", "unit dosage" or "unit" refers to a physically discrete unit that contains a predetermined quantity of active ingredient calculated to produce a desired therapeutic effect. The unit dose, unit dosage or unit may be in the form of a vial, tablet, capsule, sachet, etc. referred to herein as a "unit dosage form".

In a further embodiment of the pharmaceutical composition as herein disclosed, the pharmaceutically acceptable salt of caspofungin is an acetate salt, preferably the diacetate salt of caspofungin. An acetate salt of caspofungin, such as a diacetate salt of caspofungin may be prepared as disclosed in US 5,952,300.

In a further embodiment, it is provided a pharmaceutical composition comprising:
a) a pharmaceutically effective amount of caspofungin and/or a pharmaceutically acceptable salt thereof;
b) a pharmaceutically acceptable amount of one or more excipients effective to form a cake;
c) a pharmaceutically acceptable amount of a buffering agent selected from the group consisting of carbonic acid, bicarbonate, carbon dioxide, carbonate and mixtures thereof, and wherein the buffering agent of bicarbonate or carbonate are salts of an alkali metal or an alkaline earth metal, preferably are salts of an alkali metal, more preferably are sodium bicarbonate and/or sodium carbonate and mixtures thereof.

In a further embodiment of the pharmaceutical composition as herein disclosed, the pharmaceutically acceptable amount of a buffering agent selected from the group consisting of carbonic acid, bicarbonate, carbon dioxide, carbonate and mixtures thereof has been added during the manufacturing of said pharmaceutical composition. Suitable buffering agents of bicarbonate or carbonate are salts of an alkali metal or an alkaline earth metal, preferred are salts of an alkali metal, more preferred are sodium bicarbonate and/or sodium carbonate and mixtures thereof.

In a further embodiment of the pharmaceutical composition as herein disclosed, the buffering agent is bicarbonate and/or carbon dioxide, preferably is carbon dioxide.

The term "buffering agent" refers to one or more components which are added to a composition in order to adjust or maintain the pH of the composition. The buffering agent can be obtained by either dissolving the salt or the acid form of the buffering agent into water e.g. bicarbonate salt or carbonic acid or bubbling carbon dioxide into the solution. When preparing the composition of the present invention, the buffering agent may also be used in solid form, such as by adding bicarbonate salt into a solution of excipients. Said buffering agent is used in a pharmaceutically acceptable amount ensuring the provision and maintenance of a pharmaceutically acceptable pH value. Preferably, said buffering agent is added by bubbling carbon dioxide.

In a further embodiment of the pharmaceutical composition as herein disclosed, the molar ratio of the pharmaceutically effective amount of caspofungin and/or a pharmaceutically acceptable salt thereof and the buffering agent is from 10:1 to 1:5, preferably is from 3:1 to 1:1.

The excipient comprised in the composition of the present invention is effective to form a lyophilized cake. The term "excipient effective to form a lyophilized cake" as herein disclosed is understood to mean that this excipient adds bulk to a formulation or composition which results in a well-formed cake upon freeze drying, i.e. lyophilization. Said excipient is preferably a bulking agent.

The term "bulking agent" refers to a pharmaceutically acceptable excipient which provides bulk to the formulation such that when unit dosage amounts of the solution are lyophilized in containers, such as sterile vials, the freeze-dried residue will be clearly discernible. Acceptable bulking agents include, but are not limited to, carbohydrates such as simple sugars such as dextrose, ribose, fructose and the like, alcohol sugars such as mannitol, inositol and sorbitol, disaccharides including trehalose, sucrose and lactose, naturally occurring polymers such as starch, dextrans, proteins (e.g., gelatin and serum albumin) and glycogen, and synthetic monomers and polymers.

In a further embodiment of the pharmaceutical composition as herein disclosed, it comprises at least one bulking agent, preferably the weight ratio of the pharmaceutically effective amount of caspofungin and/or a pharmaceutically acceptable salt thereof and the total amount of bulking agents is from 1:10 to 1:0.1, more preferably 1:4 to 1:0.3, and even more preferably is from 1:3 to 1:0.5.

In a further embodiment of the pharmaceutical composition as herein disclosed, the bulking agent is selected from the group consisting of a saccharide such as glucose, mannose, galactose, ribose, trehalose, sucrose, lactose, mannitol; a polymer such as glycogen, starch, dextrans; a protein such as gelatin or serum albumin; and a mixture thereof, preferably the bulking agent is selected from sucrose, mannitol and a mixture thereof. More preferably, the bulking agent is a mixture of sucrose and mannitol.

In a further embodiment of the pharmaceutical composition as herein disclosed, the composition is in form of a solution and is suitable to form a lyophilized cake. Preferably, the solution is an aqueous solution.

The pH of the composition of the invention can be physiologically compatible and/or sufficient to maintain stability of the composition. In a further embodiment of the pharmaceutical composition as herein disclosed, said composition is in form of a solution, and the pH of the solution is from about 3.8 to about 7, preferably is from about 5.0 to about 7.0.

The determination of pH is measured by the method described in European Pharmacopoeia 7.0th edition 2.2.3. (Potentiometric determination of pH). The pH is a number which represents conventionally the hydrogen ion concentration of an aqueous solution. The potentiometric determination of pH is made by measuring the potential difference between 2 appropriate electrodes immersed in the solution to be examined. A Crison pH meter GLP21 was used. This instrument uses a pH electrode sensor A.T.C. type Pt 1000, measures electric potential differences with a high entry impedance and high resolution. The instrument was calibrated before the first measurement and after each 15 measurements with tow reference solution having pH values of 4.00 and 7.02.

In a further embodiment of the pharmaceutical composition as herein disclosed, the composition is in the form of a lyophilized cake, wherein said lyophilized cake is suitable for reconstitution to form a liquid composition for parenteral, preferably intravenous, administration.

In a further embodiment of the pharmaceutical composition as herein disclosed, the water content of the lyophilized cake is less than 3.0 % by weight of the total amount of the lyophilized cake, preferably is between 0.01 % and 1.0 % by weight of the total amount of the lyophilized cake, more preferably is between 0.05 % and 0.5 % by weight of the total amount of the lyophilized cake.

The determination of water content is measured by the method described in European Pharmacopoeia 7.0th edition 2.5.32. (Water: Micro Determination). The coulometric titration is restricted to the quantitative determination of small amounts of water.

In a further embodiment of the pharmaceutical composition as herein disclosed, the caspofungin diacetate is in amorphous form.

The composition described herein results in low formation of total impurities during storage of a lyophilized formulation prepared therefrom compared with lyophilized formulations based on an acetate buffer system or lyophilized formulations known in the art prepared from a composition not comprising a buffering agent. The term "total impurities" as used herein means the total amount of impurities commonly present in a pharmaceutically acceptable caspofungin product or a pharmaceutically active caspofungin salt prepared according to methods for preparing caspofungin or a salt thereof well known to the skilled person in the art. The total amount of impurities present may be measured by HPLC analysis. The person skilled in the art is familiar with various applicable HPLC devices and methods for measuring the formation of impurities during storage.

Once lyophilized, the pharmaceutical compositions as herein disclosed are packaged (e.g. the vials are stoppered) and ready for storing and/or shipping to end users. For use, the lyophilized cake is reconstituted by adding a suitable reconstitution solution. Then, the reconstituted composition is further diluted in a bag for intravenous administration. In a further embodiment of the pharmaceutical composition of the third aspect, the lyophilized cake of the present invention is reconstituted in a sterile aqueous solution, preferably the sterile aqueous solution is distilled and/or sterile water for injection, bacteriostatic water for injection optionally comprising methylparabene and/or propylparabene and/or 0.9 % w/w benzyl alcohol, saline, 5 % glucose solution, 5% dextrose solution, Hartmann's solution, Ringer's solution and/or lactated Ringer's solution, more preferably is saline or lactated Ringer's solution.

The composition according to the present invention is prepared by dissolving and mixing the ingredients, filtering the obtained mixture, and after transferring the solution to suitable vials. The so obtained solution is lyophilized to obtain a lyophilized cake. Lyophilization, also called freeze-drying, refers to a drying process by freezing a material dissolved in water below 0° C and then reducing the surrounding pressure using a vacuum pump to allow the frozen water in the material to sublimate directly from the solid phase to the gas phase. The composition according to the present invention is preferably lyophilized in pharmaceutically acceptable vials according to the method of the present invention to obtain a lyophilized cake of the composition of the present invention.

In a further embodiment of the process of the fourth aspect for the manufacture of a pharmaceutical composition, the steps from (i) to (iv) are carried out at a temperature below 15 °C, preferably between 2 °C and 10 °C. Preferably, the buffering agent in step (i) is carbon dioxide and the agent used in step (iii) is NaOH. More preferably, the pH is adjusted to 5.0 - 7.0 in step (iii) and even more preferably the pH is adjusted to 5.5 - 6.6 in step (iii).

In a further embodiment of the process for the manufacture of a pharmaceutical composition as herein disclosed, the pharmaceutical composition is in a vial and the vial is sealed after lyophilization under an inert atmosphere, preferably the inert atmosphere is nitrogen or argon atmosphere.

The lyophilized composition is then appropriately sealed and stored (e.g., in stoppered vials) for later use. In a further embodiment, the vial as herein disclosed has a volume between 3 and 12 ml, preferably between 4 and 11 ml. More preferably, the vial is capped with a rubber stopper and a seal.

In a further embodiment of the pharmaceutical batch of the sixth disclosure, the batch comprises at least 1,200 units, preferably at least 4,000 units.

Another aspect of the present disclosure relates to the pharmaceutical composition and the pharmaceutical batch as herein disclosed for use in the treatment of an infectious disease, preferably for use in the treatment of fungal infections or conditions caused by *Candida* species and/or *Aspergillus* species.

The term "batch" as used herein refers to a specific quantity of a drug or other material that is intended to have uniform character and quality, within specified limits, and is produced according to a single manufacturing order during the same cycle of manufacture. A batch, in the case of a drug product produced by continuous process, is a specific identified amount produced in a unit of time or quantity in a manner that assures its having uniform character and quality within specified limits (Code of Federal Regulations Title 21, Food and Drug Administration department of Health and Human Services, Subchapter C, Section 210.3 (b) (2) and (10)).

The term "pharmaceutical batch" as used herein refers to a batch as defined above of a pharmaceutical composition manufactured in accordance with the principles and guidelines of Good Manufacturing Practice (GMP) at an industrial scale and which is intended for commercialization (Directive 91/356/EEC).

The pharmaceutical composition may be manufactured at laboratory scale, not necessarily following GMP and not intended for commercialization. The pharmaceutical composition may also be manufactured for validation, following GMP. A batch of a pharmaceutical composition which is manufactured for validation is called "pilot batch".

Each pharmaceutical batch of finished product must fulfil the regulatory requirements of the corresponding Medicine Agency before being released for sale or supply, such as impurities thresholds and stability data.

The term "uniform" as used herein refers to the content of the active ingredient in the vials of a pharmaceutical batch has to be homogeneous. According to the FDA criteria, uniformity is considered as achieving 90-110 % potency of the theoretical strength with a relative standard deviation (RSD) of less than 5 % for all samples (Guidance for Industry ANDA's: Blend Uniformity Analysis, published August 1999).

The lyophilization (freeze-drying) process of the eleventh aspect for the manufacture of a lyophilized cake comprising caspofungin or a pharmaceutically acceptable salt thereof comprises at least three steps: freezing, primary drying, and secondary drying. The lyophilization process as herein disclosed provides a good quality of the resulting cake in the minimum time. This process maximizes the rate of heat transfer to each vial without causing cake collapse and avoiding slower reconstitution times. The freeze-drying cycle is efficient and robust without compromising the pharmaceutical quality of the product and neither other parameters such as stability and water content.

The term "primary drying" refers to heating the material at low temperature and low pressure to cause frozen "free water" to sublimate directly to vapour. The term "secondary drying", also known as the desorption drying, is heating at a higher temperature, so that the bound unfrozen water absorbs the heat of desorption and becomes free water; then the free water absorbs the heat of evaporation and becomes vapour escaping from the material finally.

In a further embodiment of the process for the manufacture of a lyophilized cake comprising caspofungin or a pharmaceutically acceptable salt thereof as herein disclosed, the vacuum ranges from 0.01 to 1 mBar, preferably from 0.05 to 0.5 mBar, more preferably from 0.1 to 0.2 mBar.

In a preferred embodiment of the process for the manufacture of a lyophilized cake comprising caspofungin or a pharmaceutically acceptable salt thereof as herein disclosed, the vacuum in step (ii) ranges from 0.01 to 1 mbar, preferably from 0.05 to 0.5 mbar, more preferably from 0.1 to 0.2 mbar. In a preferred embodiment, the vacuum in step (iii) ranges from 0.002 to 1 mbar, preferably from 0.004 to 0.5 mbar, more preferably from 0.005 to 0.015 mbar.

In a preferred embodiment, the temperature in step (i) is between -40 °C and -50 °C and is held between 3 hours to 6 hours; the temperature in step (ii) is between 10 °C and 0 °C, and the pressure is 0.15 mbar, and these conditions are held between 25 to 40 hours; and in step (iii) the temperature is between +35 °C to 25 °C, the pressure is 0.01 mbar, and these conditions are held for at least 4 hours.

The "suitable solution" refers to a solution suitable to form a lyophilized cake. In a further embodiment, the suitable solution comprises a pharmaceutically effective amount of caspofungin or a pharmaceutically acceptable salt thereof, preferably comprises caspofungin diacetate salt in aqueous solution.

In a further embodiment of the suitable solution as herein disclosed, the concentration of caspofungin free base present in the solution ranges from 5 to 40 mg/ml, preferably from 10 to 25 mg/ml and more preferably 15 to 20 mg/ml.

In a further embodiment of the suitable solution as herein disclosed, the volume to be filled in the vials to form a lyophilized cake ranges from 0.5 to 10 ml, preferably from 1 to 8 ml and more preferably from 2.5 to 5 ml.

In a further embodiment of the suitable solution as herein disclosed, the concentration of caspofungin free base present in the solution ranges from 10 to 25 mg/ml and the volume to be filled in the vials to form a lyophilized cake ranges from 1 to 8 ml.

In a further embodiment of the suitable solution as herein disclosed, the weight ratio of the pharmaceutically effective amount of caspofungin and/or a pharmaceutically acceptable salt thereof and the total amount of bulking agents is from 1:10 to 1:0.1, preferably from 1:4 to 1:0.3, even more preferably from 1:3 to 1:0.5.

In a further embodiment of the suitable solution as herein disclosed, the weight ratio of the pharmaceutically effective amount of caspofungin and/or a pharmaceutically acceptable salt thereof and the total amount of bulking agents is from 1:4 to 1:0.3 and the bulking agent is selected from the group consisting of a saccharide such as glucose, mannose, galactose, ribose, trehalose, sucrose, lactose, mannitol; a polymer such as glycogen, starch, dextrans; a protein such as gelatin or serum albumin; and a mixture thereof.

In a further embodiment of the suitable solution as herein disclosed, the solution is the solution as defined in the first aspect of the present invention.

In a further embodiment of the pharmaceutical composition as herein disclosed, the process of freeze drying for the pharmaceutical composition comprising about 50 mg of caspofungin as free base by unit dose is less than 48 hours long, preferably less than 40 hours long, and more preferably less than 38 hours long from the initial step of freezing the solution until the end of the secondary drying.

In a further embodiment of the pharmaceutical composition as herein disclosed, the process of freeze drying for the pharmaceutical composition comprising about 70 mg of caspofungin as free base by unit dose is less than 55 hours long, preferably less than 48 hours long from the initial step of freezing the solution until the end of the secondary drying.

Unless otherwise indicated, all the analysis methods are carried out according to the European Pharmacopoeia 7th edition.

Further aspects and embodiments disclosed herein can be found in the following numbered clauses:
Clause 1.- A pharmaceutical composition comprising:
   a) a pharmaceutically effective amount of caspofungin and/or a pharmaceutically acceptable salt thereof;
   b) a pharmaceutically acceptable amount of one or more excipients effective to form a cake;
   c) a pharmaceutically acceptable amount of a buffering agent selected from the group consisting of carbonic acid, bicarbonate, carbon dioxide, carbonate and mixtures thereof.
Clause 2.- The pharmaceutical composition according to the preceding clause, wherein the pharmaceutically acceptable amount of a buffering agent selected from the group consisting of carbonic acid, bicarbonate, carbon dioxide, carbonate and mixtures thereof has been added during the manufacturing of said pharmaceutical composition.
Clause 3.- The pharmaceutical composition according to any one of the preceding clauses, wherein the pharmaceutically acceptable salt of caspofungin is an acetate salt, preferably the diacetate salt of caspofungin.
Clause 4.- The pharmaceutical composition according to any one of the preceding clauses, wherein the pharmaceutical composition comprises between 25 and 80 mg of caspofungin as free base by unit dose.
Clause 5.- The pharmaceutical composition according to any one of the preceding clauses, wherein the pharmaceutical composition comprises about 50 mg of caspofungin as free base by unit dose or about 70 mg of caspofungin as free base by unit dose.
Clause 6.- The pharmaceutical composition according to any one of the preceding clauses, wherein the buffering agent is bicarbonate and/or carbon dioxide, preferably the buffering agent is carbon dioxide.
Clause 7.- The pharmaceutical composition according to any one of the preceding clauses, wherein the molar ratio of the pharmaceutically effective amount of caspofungin and/or a pharmaceutically acceptable salt thereof and the buffering agent is from 10:1 to 1:5, preferably from 3:1 to 1:1.
Clause 8.- The pharmaceutical composition according to any one of the preceding clauses, comprising at least one bulking agent.
Clause 9.- The pharmaceutical composition according to the preceding clause, wherein the weight ratio of the pharmaceutically effective amount of caspofungin and/or a pharmaceutically acceptable salt thereof and the total amount of bulking agents is from 1:10 to 1:0.1, preferably from 1:4 to 1:0.3, even more preferably from 1:3 to 1:0.5.
Clause 10.- The pharmaceutical composition according to any of the two preceding clauses, wherein the bulking agent is selected from the group consisting of a saccharide such as glucose, mannose, galactose, ribose, trehalose, sucrose, lactose, mannitol; a polymer such as glycogen, starch, dextrans; a protein such as gelatin or serum albumin; and a mixture thereof.
Clause 11.- The pharmaceutical composition according to any of the three preceding clauses, wherein the bulking agent is selected from sucrose, mannitol and a mixture thereof, preferably the bulking agent is a mixture of sucrose and mannitol.
Clause 12.- The pharmaceutical composition according to any one of the preceding clauses, wherein the composition is in form of a solution and is suitable to form a lyophilized cake.
Clause 13.- The pharmaceutical composition according to the preceding clause, wherein the solution is an aqueous solution.
Clause 14.- The pharmaceutical composition according to any one of the two preceding clauses, wherein the pH of the solution is from about 3.8 to about 7, preferably from about 5.0 to about 7.0.
Clause 15.- The pharmaceutical composition according to any one of the preceding clauses 1 to 12, wherein the composition is in the form of a lyophilized cake, wherein said lyophilized cake is suitable for reconstitution to form a liquid composition for parenteral, preferably intravenous, administration.
Clause 16.- The pharmaceutical composition according to the preceding clause, wherein the water content of the lyophilized cake is less than 3.0 % by weight of the total amount of the lyophilized cake, preferably is between 0.01 % and 1.0 % by weight of the total amount of the lyophilized cake.
Clause 17.- The pharmaceutical composition according to any one of the preceding clauses, wherein the caspofungin diacetate is in amorphous form.
Clause 18.- A lyophilized cake obtainable by lyophilization of the composition in form of a solution as defined in the preceding clauses, wherein said lyophilized cake is suitable for reconstitution to form a liquid composition for parenteral, preferably intravenous, administration.
Clause 19.- A pharmaceutical composition obtainable by reconstitution of the lyophilized cake as defined in any one of the preceding clauses 15 to 18 with a sterile aqueous solution.
Clause 20.- The pharmaceutical composition comprising the lyophilized cake according to any one of clauses 15 to 19 reconstituted in a sterile aqueous solution.
Clause 21.- The pharmaceutical composition according to the preceding clause, wherein the sterile aqueous solution is distilled and/or sterile water for injection, bacteriostatic water for injection optionally comprising methylparabene and/or propylparabene and/or 0.9 % w/w benzyl alcohol, saline, 5 % glucose solution, 5% dextrose solution, Hartmann's solution, Ringer's solution and/or lactated Ringer's solution, preferably the sterile aqueous solution is saline or lactated Ringer's solution.
Clause 22.- A process for the manufacture of a pharmaceutical composition as defined in any one of the preceding clauses, comprising at least the following steps:
   i) preparing an aqueous solution comprising a pharmaceutically acceptable amount of one or more excipients with a pharmaceutically acceptable amount of the buffering agent;
   ii) adding to the solution of (i) a pharmaceutically acceptable amount of caspofungin and/or a pharmaceutically acceptable salt thereof;
   iii) optionally, adjusting the pH between 4 and 7;
   iv) filtering the solution obtained in the previous step;
   v) freezing the solution obtained in step (iv); and
   vi) freeze drying the frozen solution obtained in step (v).
Clause 23.- The process according to any of the preceding clauses, wherein the steps from (i) to (iv) are carried out at a temperature below 15 °C, preferably between 2 and 10 °C.
Clause 24.- The process according to any of the two preceding clauses, wherein the buffering agent in step (i) is carbon dioxide and the agent used in step (iii) is NaOH.
Clause 25.- The process according to any of the three preceding clauses, wherein the pH is adjusted to 5.0 - 7.0 in step (iii), preferably the pH is adjusted to 5.5 - 6.6 in step (iii).
Clause 26.- The process according to any of the four preceding clauses, wherein the pharmaceutical composition is in a vial and the vial is sealed after lyophilization under an inert atmosphere, preferably the inert atmosphere is nitrogen or argon atmosphere.
Clause 27.- A pharmaceutical composition obtained by the process as defined in any one of the preceding process clauses.
Clause 28.- A pharmaceutical batch comprising any one of the preceding pharmaceutical compositions as defined in any of the preceding clauses.
Clause 29.- A pharmaceutical batch comprising at least 20 % w/w of caspofungin diacetate in respect of the total amount of the batch and wherein the molar ratio of the pharmaceutically effective amount of caspofungin and/or a pharmaceutically acceptable salt thereof and the buffering agent is from 10:1 to 1:5.
Clause 30.- The pharmaceutical batch according to any one of the two preceding clauses, comprising at least 1,200 units, preferably comprising at least 4,000 units.
Clause 31.- The pharmaceutical composition as defined in any one of clauses 1 to 21 or in clause 27, or the pharmaceutical batch as defined in any one of clauses 28 to 30, for use in the treatment of an infectious disease.
Clause 32.- The pharmaceutical composition as defined in any one of clauses 1 to 21 or in clause 27, or the pharmaceutical batch defined in any one of clauses 28 to 30, for use in the treatment of fungal infections or conditions caused by *Candida* species and/or *Aspergillus* species.
Clause 33.- A glass vial comprising the pharmaceutical composition as defined in any one of clauses 1 to 27 or in clause 27.
Clause 34.- The vial according to the preceding clause, wherein the volume of said vial is between 3 and 12 ml, preferably between 4 and 11 ml.
Clause 35.- The vial according to any one of the two preceding clauses, wherein said vial is capped with a rubber stopper and a seal.
Clause 36.- A cardboard box with a patient information leaflet comprising at least one unit of the pharmaceutical composition as defined in any one of clauses 1 to 21 or in clause 27 or at least one vial as defined in any one of clauses 33 to 35.
Clause 37.- A process for the manufacture of a lyophilized cake comprising caspofungin or a pharmaceutically acceptable salt thereof, wherein the process of freeze drying is less than 96 hours long, preferably less than 72 hours long, and more preferably less than 48 hours long from the initial step of freezing the solution until the end of the secondary drying.
Clause 38.- The process according to the preceding clause comprising at least the following steps:
   i) freezing a suitable solution to a first temperature, wherein the first temperature is a temperature between -40 °C and -50 °C, and hold between at least 3 hours to 8 hours at the first temperature;
   ii) primary drying between 20 to 40 hours under vacuum from the first temperature to a second temperature, wherein the second temperature is between 10 °C and 0 °C;
   iii) secondary drying for at least 4 hours under vacuum from the second temperature to a third temperature, wherein the third temperature is between +35 °C to +25 °C.

All percentages, parts and ratios herein disclosed are by weight unless specifically noted otherwise. As used herein, the term "about" refers preferably to a range that is +-10 %, preferably +-5 %, or more preferably the value with which the term is associated.

### DESCRIPTION OF THE FIGURE

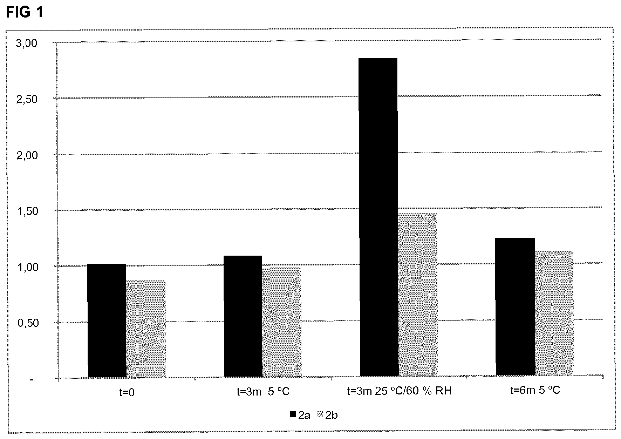

Figure 1 shows the change in the amount of the total impurities (%) in the lyophilized formulations prepared according to example 2 after 3 months (t=3) and 6 months (t=6) storage, respectively, i.e. caspofungin composition comprising caspofungin diacetate, sucrose, mannitol and as buffering agent either carbon dioxide or acetate.

### EXAMPLES

The following examples illustrate various embodiments of the invention and are not intended to limit the invention in any way:

### Example 1: Preparation of caspofungin diacetate formulations.

A caspofungin composition was prepared by firstly dissolving mannitol and sucrose or trehalose/kollidon in water. The solution was stirred for about 30 minutes until complete dissolution of the excipients. The solution was bubbled with 0.03 equivalents of carbon dioxide until pH was 4.30. After the addition of carbon dioxide, the solution was kept at 4-6 °C. To the so obtained solution, caspofungin diacetate corresponding to 70 mg/vial caspofungin was added. The volume of the solution was adjusted to 15 ml by adding water acceptable for injection. The pH of the obtained solution was then again adjusted to pH 6.6 with 0.1 M NaOH, and the solution was stirred.

The solution was filtered through a 0.2 *µ*m pore size syringe filter. The solution was thereafter transferred to 10 ml lyophilization vials and pre-stopped with sterile rubber stoppers. The solution was then subjected to lyophilization. The preparation of the composition, except during freeze drying, was performed at a temperature of 4-6 °C.

The amount of the various ingredients of the composition was as listed in table 1 below.

**Table 1**

| | **Ex1a** | **Ex.1b** | **Ex. 1c** | **Ex.1d** |
|---|---|---|---|---|
| Caspofungin diacetate (mg/vial) | 77.70 | 77.70 | 77.70 | 77.70 |
| Mannitol (mg/vial) | 84.0 | 84.0 | 84.0 | 84.0 |
| Sucrose (mg/vial) | | 125.0 | | 125.0 |
| Trehalose (mg/vial) | 125.0 | | 125.0 | |
| Kollidon K17 (mg/vial) | 25.0 | | 25.0 | |
| Acetic acid ⁽²⁾ | | | | q.s. to pH 3.0 |
| Carbon dioxide ⁽²⁾ | q.s to pH 3.8 - 4.5 | q.s to pH 3.8 - 4.5 | | q.s. to pH 3.8 - 4.5 |
| NaOH 0.1 M ⁽¹⁾ | | to pH 5.1 | | to pH 5.1 |
| Purified water ⁽¹⁾ | q.s. to 1 ml | q.s. to 1 ml | q.s. to 1 ml | q.s. to 1 ml |

| | | | | |
|---|---|---|---|---|
| (1) "q.s." means adding a quantity sufficient to achieve a certain state (e.g., volume, i.e., to bring a solution to a desired volume). (2) "q.s. to pH" means the addition of acid or base in a quantity sufficient to bring a solution to a desired pH (e.g., q.s. to pH 4.5 means the addition of acid or base to bring the solution to a pH of 4.5). | | | | |

### Example 2: Preparation of caspofungin diacetate formulations.

The caspofungin compositions of Example 2 were prepared analogously to Example 1. Both compositions were prepared at the same time and subjected to the same lyophilization cycle in order to minimize external factors that could affect the final formula.

The amount of the various ingredients of the composition was as listed in table 2 below.

**Table 2**

| | **Ex2a** | **Ex.2b** | **Ex.2c**⁽³⁾ |
|---|---|---|---|
| Caspofungin diacetate (mg/vial) | 77.70 | 77.70 | 77.70 |
| Mannitol (mg/vial) | 36.4 | 36.4 | 33.3 |
| Sucrose (mg/vial) | 54.6 | 54.6 | 50.0 |
| Acetic acid (mg/vial) ⁽²⁾ | 2.8 | | |
| Carbon dioxide ⁽²⁾ | | q.s to pH 3.8 - 4.5 | q.s to pH 3.8 - 4.5 |
| NaOH 0.1M ⁽¹⁾ | | q.s to pH 6.6 | q.s to pH 6.6 |
| Purified water ⁽¹⁾ | q.s. to 1 ml | q.s. to 1 ml | q.s. to 3.9 ml ⁽³⁾ |

| | | | |
|---|---|---|---|
| (1) "q.s." means adding a quantity sufficient to achieve a certain state (e.g., volume, i.e., to bring a solution to a desired volume). (2) "q.s. to pH" means the addition of acid or base in a quantity sufficient to bring a solution to a desired pH (e.g., q.s. to pH 4.5 means the addition of acid or base to bring the solution to a pH of 4.5). (3) Vials were overfilled with 8% volume to fill the vials with 4.2 ml of the filtered solution | | | |

### Lyophilization process

The solutions prepared either in Example 1 or 2 were subjected to lyophilization. Two batches of 350 vials/batch were lyophilized following the process for the manufacture of a lyophilized cake comprising caspofungin described in the embodiments of the description of the eleventh aspect.

### Example 3. Stability Studies for formulations disclosed in Example 1 and 2 - Impurity profile.

The formulations prepared according to Example 1 and Example 2 were stored in the lyophilized state at 5 °C and 25 °C and 60 % of relative humidity (RH) for 3 months and 6 months, respectively, before stability testing. Prior to testing, the lyophilized material was dissolved in a pharmaceutically acceptable reconstitution solution, specifically sterile water for injection. The so obtained solutions were then analysed by HPLC according to standard methods well known to the skilled person in the art.

| **Ex. 1a** | **5 °C - t=0** | **5 °C** - **3 month** | **5 °C - 6 month** | **25°C / 60% RH-3 month** | **25 °C / 60% RH- 6 month** |
|---|---|---|---|---|---|
| **Water content (%)** | 0.4 | 0.2 | 0.3 | 0.2 | 0.5 |
| **Assay (%)** | 97.7 | 98.6 | 98.6 | 95.3 | 92.9 |
| **Total impurities (%)** | 1.18 | 1.00 | 1.37 | 1.97 | 2.88 |

| **Ex. 1b** | **5 °C - t=0** | **5 °C - 3 month** | **5 °C - 6 month** | **25 °C / 60% RH-3 month** | **25 °C / 60% RH-6 month** |
|---|---|---|---|---|---|
| **Water content (%)** | 0.1 | 0.2 | 0.2 | 0.2 | 0.3 |
| **Assay (%)** | 98.8 | 98.6 | 100.9 | 95.3 | 100.7 |
| **Total impurities (%)** | 1.13 | 1.25 | 1.04 | 1.97 | 1.711 |

| **Ex. 1c** | **5 °C - t=0** | **5 °C - 3 month** | **5 °C - 6 month** | **25 °C / 60% RH-3 month** | **25°C/60% RH- 6 month** |
|---|---|---|---|---|---|
| **Water content (%)** | 0.1 | 0.2 | 0.3 | 0.2 | 0.5 |
| **Assay (%)** | 97.3 | 95.2 | 99.4 | 92.0 | 96.5 |
| **Total impurities (%)** | 1.17 | 1.21 | 1.13 | 1.91 | 2.29 |

| **Ex. 1d** | **5 °C - t=0** | **5 °C - 3 month** | **5 °C - 6 month** | **25 °C / 60% RH-3 month** | **25°C/60% RH- 6 month** |
|---|---|---|---|---|---|
| **Water content (%)** | 0.2 | 0.3 | 0.3 | 0.2 | 0.3 |
| **Assay (%)** | 98.7 | 93.6 | 100.7 | 92.4 | 98.9 |
| **Total impurities (%)** | 1.17 | 1.15 | 1.17 | 1.54 | 1.74 |

The results of the stability testing, including 3 and 6 months data, of the compositions prepared according to example 2 are shown in figure 1.

| **Ex. 2a** | **5 °C - t=0** | **5 °C - 3 month** | **5 °C - 6 month** | **25 °C /60% RH-3 month** |
|---|---|---|---|---|
| **Water content (%)** | 0.2 | 0.3 | - | 0.4 |
| **Assay (%)** | 101.9 | 101.2 | 103.8 | 101.8 |
| **Total impurities (%)** | 1.02 | 1.09 | 1.23 | 2.84 |

| **Ex. 2b** | **5 °C - t=0** | **5 °C - 3 month** | **5 °C - 6 month** | **25 °C / 60% RH-3 month** |
|---|---|---|---|---|
| **Water content (%)** | 0.2 | 0.4 | - | 0.4 |
| **Assay (%)** | 94.6 | 101.4 | 101.0 | 100.9 |
| **Total impurities (%)** | 0.86 | 0.98 | 1.11 | 1.46 |

| **Ex. 2c** | **5 °C - t=0** |
|---|---|
| **Water content (%)** | 0.3 |
| **Assay (%)** | 106.3 |
| **Total impurities (%)** | 0.71 |

Surprisingly, the stability testing according to figure 1 revealed that a lyophilized formulation based on a composition prepared using carbon dioxide as a buffering agent showed lower formation of total impurities compared with the composition comprising acetate. The fact that acetate seems not to be superior in respect of formation of the impurities determined is surprising taking into account the teaching of the prior art. As mentioned above, US 5,952,300 teach that the use of tartrate buffer results in the formation of undesired degradation product in contrast to acetate buffer.

## Claims

1. A pharmaceutical composition comprising:
a) a pharmaceutically effective amount of caspofungin and/or a pharmaceutically acceptable salt thereof;
b) a pharmaceutically acceptable amount of one or more excipients effective to form a cake;
c) a pharmaceutically acceptable amount of a buffering agent selected from the group consisting of carbonic acid, bicarbonate, carbon dioxide, carbonate and mixtures thereof.

2. The pharmaceutical composition according to claim 1, wherein said pharmaceutically acceptable salt of caspofungin is the diacetate salt of caspofungin and wherein the composition comprises a pharmaceutically acceptable amount of a buffering agent selected from the group consisting of carbonic acid, bicarbonate, carbon dioxide and mixtures thereof, preferably bicarbonate.

3. The pharmaceutical composition according to any one of the preceding claims, wherein the molar ratio of the pharmaceutically effective amount of caspofungin and/or a pharmaceutically acceptable salt thereof and the buffering agent is from 10:1 to 1:5.

4. The pharmaceutical composition according to any one of the preceding claims, comprising at least one bulking agent, preferably the bulking agent is selected from the group consisting of a saccharide, a polymer, a protein and mixtures thereof.

5. The pharmaceutical composition according to claim 4 wherein said saccharide is glucose, mannose, galactose, ribose, trehalose, sucrose, lactose or mannitol, said polymer is glycogen, starch or a dextrans and said protein is gelatin or serum albumin, preferably said bulking agent comprises sucrose and mannitol.

6. The pharmaceutical composition according to any one of the preceding claims, wherein the composition is in the form of a lyophilized cake, wherein said lyophilized cake is suitable for reconstitution to form a liquid composition for parenteral, preferably intravenous, administration.

7. The pharmaceutical composition according to claim 6, wherein the water content of the lyophilized cake is less than 3.0 % by weight of the total amount of the lyophilized cake.

8. A lyophilized cake obtainable by lyophilization of the pharmaceutical composition in form of a solution as defined in any one of claims 1 to 5, wherein said lyophilized cake is suitable for reconstitution to form a liquid composition for parenteral, preferably intravenous, administration.

9. A pharmaceutical composition obtainable by reconstitution of the lyophilized cake according to claims 6 to 8 with a sterile aqueous solution.

10. The pharmaceutical composition according to claim 9, wherein said sterile aqueous solution is distilled and/or sterile water for injection, bacteriostatic water for injection, water comprising methylparabene and/or propylparabene and/or 0.9 % w/w benzyl alcohol, saline, 5 % glucose solution, 5% dextrose solution, Hartmann's solution, Ringer's solution and/or lactated Ringer's solution.

11. A process for the manufacture of a pharmaceutical composition as defined in any one of the preceding claims, comprising the steps of:
i) preparing an aqueous solution comprising a pharmaceutically acceptable amount of one or more excipients with a pharmaceutically acceptable amount of the buffering agent;
ii) adding to the solution of i) a pharmaceutically acceptable amount of caspofungin and/or a pharmaceutically acceptable salt thereof;
iii) optionally, adjusting the pH between 4 and 7;
iv) filtering the solution obtained in the previous step;
v) freezing the solution obtained in step iv); and
vi) freeze drying the frozen solution obtained in step v).

12. The process according to claim 11, wherein said buffering agent in step i) is carbon dioxide and the agent used in step iii) is NaOH.

13. A sealed glass vial comprising the pharmaceutical composition as defined in any one of claims 1 to 10.

14. The pharmaceutical composition according to any one of claims 1 to 10 or the glass vial according to claim 13, for use in the treatment of fungal infections or conditions caused by Candida species and/or Aspergillus species.

15. A process for the manufacture of a lyophilized cake comprising caspofungin or a pharmaceutically acceptable salt thereof, wherein the process of freeze drying is less than 96 hours long, preferably less than 72 hours long, from the initial step of freezing the solution until the end of the secondary drying, wherein the process comprises at least the following steps:
i) freezing a suitable solution to a first temperature, wherein the first temperature is a temperature between -40 °C and -50 °C, and holding for between at least 3 hours to 8 hours at the first temperature;
ii) primary drying between 20 to 40 hours under vacuum from the first temperature to a second temperature, wherein the second temperature is between 10 °C and 0 °C;
iii) secondary drying for at least 4 hours under vacuum from the second temperature to a third temperature, wherein the third temperature is between +35 °C to +25 °C.

## Patentansprüche

1. Pharmazeutische Zusammensetzung, umfassend:
a) eine pharmazeutisch verträgliche Menge von Caspofungin und/oder einem pharmazeutisch verträglichen Salz davon;
b) eine pharmazeutisch verträgliche Menge eines oder mehrerer Hilfsstoffe, die zum Bilden eines Kuchens wirksam sind;
c) eine pharmazeutisch verträgliche Menge eines Puffers, ausgewählt aus der Gruppe, bestehend aus Kohlensäure, Bicarbonat, Kohlendioxid, Carbonat und Mischungen davon.

2. Pharmazeutische Zusammensetzung nach Anspruch 1, wobei das pharmazeutisch verträgliche Salz von Caspofungin das Diacetatsaltz von Caspofungin ist und wobei die Zusammensetzung eine pharmazeutisch verträgliche Menge eines Puffers umfasst, der ausgewählt ist aus der Gruppe, bestehend aus Kohlensäure, Bicarbonat, Kohlendioxid und Mischungen davon, vorzugsweise Bicarbonat.

3. Pharmazeutische Zusammensetzung nach einem der vorhergehenden Ansprüche, wobei das Molverhältnis der pharmazeutisch verträglichen Menge von Caspofungin und/oder einem pharmazeutisch verträglichen Salz davon und dem Puffer von 10:1 bis 1:5 beträgt.

4. Pharmazeutische Zusammensetzung nach einem der vorhergehenden Ansprüche, umfassend mindestens einen Füllstoff, wobei der Füllstoff vorzugsweise ausgewählt ist aus der Gruppe, bestehend aus einem Saccharid, einem Polymer, einem Protein und Mischungen davon.

5. Pharmazeutische Zusammensetzung nach Anspruch 4, wobei das Saccharid Glucose, Mannose, Galactose, Ribose, Trehalose, Saccharose, Lactose oder Mannitol ist, das Polymer Glycogen, Stärke oder ein Dextran ist und das Protein Gelatine oder Serumalbumin ist, wobei der Füllstoff vorzugsweise Saccharose und Mannitol umfasst.

6. Pharmazeutische Zusammensetzung nach einem der vorhergehenden Ansprüche, wobei die Zusammensetzung in Form eines lyophilisierten Kuchens vorliegt, wobei der lyophilisierte Kuchen zur Rekonstituierung zum Bilden einer Flüssigkeitszusammensetzung zur parenteralen, vorzugsweise intravenösen, Verabreichung geeignet ist.

7. Pharmazeutische Zusammensetzung nach Anspruch 6, wobei der Wassergehalt des lyophilisierten Kuchens kleiner als 3,0 Gew.-% der Gesamtmenge des lyophilisierten Kuchens beträgt.

8. Lyophilisierter Kuchen, erhältlich durch Lyophilisierung der pharmazeutischen Zusammensetzung in Form einer Lösung wie in einem der Ansprüche 1 bis 5 definiert, wobei der lyophilisierte Kuchen zur Rekonstituierung zum Bilden einer Flüssigkeitszusammensetzung zur parenteralen, vorzugsweise intravenösen, Verabreichung geeignet ist.

9. Pharmazeutische Zusammensetzung, erhältlich durch die Rekonstituierung des lyophilisierten Kuchens nach einem der Ansprüche 6 bis 8 mit einer sterilen wässrigen Lösung.

10. Pharmazeutische Zusammensetzung nach Anspruch 9, wobei die sterile wässrige Lösung destilliertes und/oder steriles Wasser zur Injektion, bakteriostatisches Wasser zur Injektion, Wasser, umfassend Methylparaben und/oder Propylparaben und/oder 0,9 % Gew./Gew. Benzylalkohol, Salzlösung, 5 %-ige Glucoselösung, 5 %-ige Dextroselösung, Hartmannslösung, Ringerlösung und/oder Ringer-Lactat-Lösung ist.

11. Verfahren zur Herstellung einer pharmazeutischen Zusammensetzung wie in einem der vorhergehenden Ansprüche definiert, umfassend die folgenden Schritte:
i) Herstellen einer wässrigen Lösung, umfassend eine pharmazeutisch verträgliche Menge eines oder mehrerer Hilfsstoffe mit einer pharmazeutisch verträglichen Menge eines Puffers;
ii) Zugeben zu der Lösung von i) einer pharmazeutisch verträglichen Menge von Caspofungin und/oder einem pharmazeutisch verträglichen Salz davon;
iii) wahlweise, Einstellen des pH-Werts zwischen 4 und 7;
iv) Filtern der Lösung, erhalten in dem vorherigen Schritt;
v) Einfrieren der Lösung, die in Schritt iv) erhalten wurde; und
vi) Gefriertrocknen der gefrorenen Lösung, die in Schritt v) erhalten wurde.

12. Verfahren nach Anspruch 11, wobei der Puffer in Schritt i) Kohlendioxid, ist und das Mittel, das in Schritt iii) verwendet wird, NaOH ist.

13. Versiegelte Glasflasche, umfassend die pharmazeutische Zusammensetzung wie in einem der Ansprüche 1 bis 10 definiert.

14. Pharmazeutische Zusammensetzung nach einem der Ansprüche 1 bis 10 oder Glasflasche nach Anspruch 13, für die Verwendung bei der Behandlung von Pilzinfektionen oder -zuständen, die durch Candida-Spezies und/oder Aspergillus-Spezies verursacht werden.

15. Verfahren zur Herstellung eines lyophilisierten Kuchens, umfassend Caspofungin oder ein pharmazeutisch verträgliches Salz davon, wobei das Gefriertrocknungsverfahren weniger als 96 Stunden andauert, vorzugsweise weniger als 72 Stunden, von dem anfänglichen Schritt des Gefrierens der Lösung bis zum Ende des sekundären Trocknens, wobei das Verfahren mindestens die folgenden Schritte umfasst:
i) Gefrieren einer geeigneten Lösung auf eine erste Temperatur, wobei die erste Temperatur eine Temperatur zwischen -40 °C und -50 °C ist, und Halten für zwischen mindestens 3 Stunden bis 8 Stunden auf einer ersten Temperatur;
ii) primäres Trocknen zwischen 20 bis 40 Stunden im Vakuum von der ersten Temperatur zu einer zweiten Temperatur, wobei die zweite Temperatur zwischen 10 °C und 0 °C beträgt;
iii) sekundäres Trocknen für mindestens 4 Stunden im Vakuum von der zweiten Temperatur zu einer dritten Temperatur, wobei die dritte Temperatur zwischen +35 °C bis +25 °C beträgt.

## Revendications

1. Composition pharmaceutique comprenant :
a) une quantité pharmaceutiquement efficace de caspofungine et/ou un sel pharmaceutiquement acceptable de celle-ci ;
b) une quantité pharmaceutiquement acceptable d'un ou de plusieurs excipients efficaces pour former un gâteau ;
c) une quantité pharmaceutiquement acceptable d'un agent tampon choisi dans le groupe constitué d'acide carbonique, de bicarbonate, de dioxyde de carbone, de carbonate et de leurs mélanges.

2. Composition pharmaceutique selon la revendication 1, dans laquelle ledit sel pharmaceutiquement acceptable de caspofungine est le sel de diacétate de caspofungine et dans laquelle la composition comprend une quantité pharmaceutiquement acceptable d'un agent tampon choisi dans le groupe constitué d'acide carbonique, de bicarbonate, de dioxyde de carbone et de leurs mélanges, de préférence de bicarbonate.

3. Composition pharmaceutique selon l'une quelconque des revendications précédentes, dans laquelle le rapport molaire de la quantité pharmaceutiquement efficace de caspofungine et /ou d'un sel pharmaceutiquement acceptable de celle-ci et de l'agent tampon est de 10:1 à 1:5.

4. Composition pharmaceutique selon l'une quelconque des revendications précédentes, comprenant au moins un agent de charge, de préférence l'agent de charge est choisi dans le groupe constitué d'un saccharide, d'un polymère, d'une protéine et de leurs mélanges.

5. Composition pharmaceutique selon la revendication 4 dans laquelle ledit saccharide est le glucose, le mannose, le galactose, le ribose, le tréhalose, le saccharose, le lactose ou le mannitol, ledit polymère est le glycogène, l'amidon ou un dextrane et ladite protéine est la gélatine ou l'albumine sérique, de préférence, ledit agent de charge comprend du saccharose et du mannitol.

6. Composition pharmaceutique selon l'une quelconque des revendications précédentes, dans laquelle la composition se présente sous la forme d'un gâteau lyophilisé, dans laquelle ledit gâteau lyophilisé est approprié pour une reconstitution pour former une composition liquide pour une administration parentérale, de préférence intraveineuse.

7. Composition pharmaceutique selon la revendication 6, dans laquelle la teneur en eau du gâteau lyophilisé est inférieure à 3,0 % en poids de la quantité totale du gâteau lyophilisé.

8. Gâteau lyophilisé pouvant être obtenu par lyophilisation de la composition pharmaceutique sous forme d'une solution telle que définie dans l'une quelconque des revendications 1 à 5, dans laquelle ledit gâteau lyophilisé est approprié pour une reconstitution pour former une composition liquide pour une administration parentérale, de préférence intraveineuse.

9. Composition pharmaceutique pouvant être obtenue par reconstitution du gâteau lyophilisé selon les revendications 6 à 8 avec une solution aqueuse stérile.

10. Composition pharmaceutique selon la revendication 9, dans laquelle ladite solution aqueuse stérile est de l'eau distillée et/ou stérile pour injection, de l'eau bactériostatique pour injection, de l'eau comprenant du méthylparabène et/ou du propylparabène et/ou 0,9 % p/p d'alcool benzylique, une solution saline, 5 % de solution de glucose, 5 % de solution de dextrose, une solution de Hartmann, une solution de Ringer et/ou une solution de Ringer lactée.

11. Procédé de fabrication d'une composition pharmaceutique telle que définie dans l'une quelconque des revendications précédentes, comprenant les étapes consistant à :
i) préparer une solution aqueuse comprenant une quantité pharmaceutiquement acceptable d'un ou de plusieurs excipients avec une quantité pharmaceutiquement acceptable de l'agent tampon ;
ii) ajouter à la solution de i) une quantité pharmaceutiquement acceptable de caspofungine et/ou d'un sel pharmaceutiquement acceptable de celle-ci ;
iii) éventuellement, ajuster le pH entre 4 et 7 ;
iv) filtrer la solution obtenue à l'étape précédente ;
v) congeler la solution obtenue à l'étape iv) ; et
vi) lyophiliser la solution congelée obtenue à l'étape v).

12. Procédé selon la revendication 11, dans lequel ledit agent tampon à l'étape i) est le dioxyde de carbone et l'agent utilisé à l'étape iii) est le NaOH.

13. Flacon en verre scellé comprenant la composition pharmaceutique telle que définie dans l'une quelconque des revendications 1 à 10.

14. Composition pharmaceutique selon l'une quelconque des revendications 1 à 10 ou flacon de verre selon la revendication 13, pour une utilisation dans le traitement d'infections fongiques ou d'affections causées par les espèces Candida et/ou les espèces Aspergillus.

15. Procédé de fabrication d'un gâteau lyophilisé comprenant de la caspofungine ou un sel pharmaceutiquement acceptable de celle-ci, dans lequel le procédé de lyophilisation dure moins de 96 heures, de préférence moins de 72 heures, de l'étape initiale de congélation de la solution jusqu'à la fin du séchage secondaire, dans lequel le procédé comprend au moins les étapes suivantes :
i) la congélation d'une solution appropriée à une première température, dans lequel la première température est une température entre -40 °C et -50 °C, et le maintien pendant au moins 3 heures à 8 heures à la première température ;
ii) le séchage primaire entre 20 et 40 heures sous vide de la première température à une deuxième température, dans lequel la deuxième température est entre 10 °C et 0 °C ;
iii) le séchage secondaire pendant au moins 4 heures sous vide de la deuxième température à une troisième température, dans lequel la troisième température est entre +35 °C et +25 °C.
